# EUROPÄISCHE PATENTSCHRIFT

(11) **EP 1 490 000 B1**
(45) Veröffentlichungstag und Bekanntmachung des Hinweises auf die Patenterteilung: **16.05.2007**
(21) Anmeldenummer: 02708118.1
(22) Anmeldetag: 04.04.2002
(51) Int. Cl.: A61F 2/44

(54) **BANDSCHEIBENPROTHESE ODER NUKLEUS-ERSATZ-PROTHESE**
INTERVERTEBRAL PROSTHESIS OR NUCLEUS REPLACEMENT PROSTHESIS
PROTHESE DISCALE OU PROTHESE DE NUCLEUS

(43) Veröffentlichungstag der Anmeldung: 29.12.2004
(73) Patentinhaber: Synthes GmbH, 4436 Oberdorf (CH)
(72) Erfinder: STUDER, Armin, CH-4513 Langendorf (CH)
(74) Vertreter: Lusuardi, Werther
(86) Internationale Anmeldenummer: PCT/CH2002/000189
(87) Internationale Veröffentlichungsnummer: WO 2003/084444

(56) Entgegenhaltungen:
- EP-A- 0 773 008
- EP-A- 1 157 676
- WO-A-02/17824
- FR-A- 2 712 486

## Beschreibung

Die Erfindung betrifft eine Bandscheibenprothese oder Nukleus-Ersatz-Prothese gemäss dem Oberbegriff des Patentanspruchs 1.

Solche Prothesen werden nach Entfernung der beschädigten, natürlichen Bandscheibe oder des beschädigten Nukleus einer Bandscheibe in den Zwischenwirbelraum zweier benachbarter Wirbelkörper eingebracht. Dabei besteht das Ziel, wieder möglichst natürliche Zustände herbeizuführen, d.h. insbesondere wieder die ursprüngliche Bandscheibenhöhe und damit den ursprünglichen Abstand zwischen den beiden benachbarten Wirbelkörpern wiederherzustellen.

Aus dem Stand der Technik sind bereits Bandscheibenprothesen bekannt, so z.B. aus der FR-A-2 712 486 BRESLAVE, bei welcher ein biegbares, aber nicht formelastisches VELKRO-Band spiralförmig zu einer Kreisscheibe aufgewickelt ist. Um die spiralförmige Aufwicklung des Bandes zu ermöglichen benötigt diese bekannte Prothese ein zylindrisches Mittelstück, an welchem das Band befestigt wird und dann durch Rotation des Mittelstücks darauf aufgewickelt wird.

Nachteilig bei dieser bekannten Bandscheibenprothese ist der Übergang zwischen dem relativ grossen Mittelstück und dem relativ schmalen Band sowie die fehlende Formelastizität des Bandes. Die Grösse des Mittelstücks bestimmt auch gleichzeitig die Grösse der Eintrittsöffnung, wobei letztere so klein als möglich gehalten werden sollte, was aber bei dieser bekannten Prothese nicht möglich ist.

Aus der EP-A-0 773 008 ist eine Zwischenwirbelprothese gemäss dem Oberbegriff des Anspruchs 1 bekannt. Nachteilig bei dieser bekannten Prothese ist wiederum das relativ grosse zylinderförmige Mittelstück an welchem der längliche, spiralförmige Körper über ein als Gelenk wirkendes Übergangsglied geringerer Breite befestigt ist. Das zylinderförmige Mittelstück mit dem nachfolgenden gelenkigen Übergangsglied ist, weil es eben federnd gelenkig angeformt ist, schwierig und aufwendig zu handhaben. Das zylinderförmige Mittelstück ist zudem hinderlich.

Die obenstehende Diskussion des Standes der Technik erfolgt lediglich zur Erläuterung des Umfeldes der Erfindung und bedeutet nicht, dass der zitierte Stand der Technik zum Zeitpunkt dieser Anmeldung oder ihrer Priorität auch tatsächlich publiziert oder öffentlich bekannt war.

Hier will die Erfindung Abhilfe schaffen. Der Erfindung liegt das Problem zugrunde, eine Bandscheibenprothese oder Nukleus-Ersatz-Prothese zu schaffen, welche dank ihrer geometrischen Form die Bandscheibenhöhe restauriert, auftretende Belastungskräfte auf der ganzen - vorteilhafterweise konvex gestalteten - Oberfläche aufnimmt und den Druck in den Facettengelenken entlastet, Kräfte in den Anulus ableitet und die natürliche Bewegung nicht beeinträchtigt, sondern abstützt.

Durch das verkleinerte innere Ende der Bandscheibenprothese oder Nukleus-Ersatz-Prothese wird eine vereinfachte Handhabung des Instrumentariums ermöglicht. Der Widerstand beim Einziehen des Implantates in das Einführungsinstrument wird dadurch geringer.
Da die Endplattenmitte sehr dünn ist, kann sie relativ einfach eingedrückt werden; dank des dünneren Querschnitts im Zentrum des Implantates ist dieses als relativ flexible Zone ausgebildet, so dass die auftretenden Drücke besser absorbiert werden.

Die Erfindung löst die gestellte Aufgabe mit einer Bandscheibenprothese, welche die Merkmale des Anspruchs 1 aufweist.

Die Verkleinerung des zur Zentralachse orthogonalen Querschnitts erfolgt vorzugsweise kontinuierlich und bevorzugt gegen das erste, äussere Ende des Körpers hin. Die senkrecht zur Zentralachse gemessene Breite des Körpers sollte sich - von seiner Mitte aus gesehen - ebenfalls gegen das äussere Ende hin - vorzugsweise kontinuierlich - verringern. Zusätzlich kann sich die Breite auch gegen das innere Ende hin, vorzugsweise kontinuierlich verringern. Dadurch wird eine erhöhte Flexibilität für das Instrumentarium zur Handhabung des Implantates erreicht.

Die Breite des Körpers ist in seiner Mitte typischerweise 50 % bis 500 %, vorzugsweise 100 % bis 300 % breiter als an seinem inneren und äusseren Ende. Dadurch kann die individuelle Flexibilität angesteuert werden und man erhält eine grössere Auflagefläche zu den Deckplatten der Wirbelkörper.

Bei einer speziellen Ausführungsform weist der Körper im spiralförmig aufgewickelten Zustand zur Zentralachse eine obere Spiralfläche und eine untere Spiralfläche auf, welche beide konvex gewölbt und zur Anlage an die Deckplatten zweier benachbarter Wirbelkörper geeignet sind. Damit wird eine Selbstzentrierung des Implantates in den konkaven Endplatten der Wirbelkörper und Erhöhung der Auflagefläche sowie eine Verringerung der spezifischen Flächenpressung erzielt. Insgesamt ergibt sich eine bessere Kräfteeinleitung in den Anulus und die Endplatte.

Zweckmässigerweise weist der Körper im spiralförmig aufgewickelten, nicht belasteten Zustand zwischen den Spiralen einen Spalt auf, was einerseits die Produktion des Körpers erleichtert und anderseits eine optimale Flexibilität garantiert. Der Spalt sollte eine Breite von mindestens 0,4 mm, vorzugsweise mindestens 0,5 mm aufweisen. Anderseits sollte der Spalt höchstens 1,0 mm, vorzugsweise höchstens 0,8 mm breit sein. Innerhalb dieser Grenzen ergibt sich ein optimaler Memory-Effekt des spiralförmig aufgewickelten Körpers.

Bei einer speziellen Ausführungsform weist der Körper im spiralförmig aufgewickelten Zustand, in der Spiralebene gesehen eine ovale oder nierenförmige Gestalt auf, vorzugsweise mit einer in der Spiralebene gemessenen Fläche von 250 bis 750 mm², was eine optimale Anpassung an die anatomischen Randbedingungen ergibt.

Bei einer bevorzugten Ausführungsform enthält der Körper ein Hydrogel oder besteht sogar ausschliesslich aus einem Hydrogel. Hydrogele sind Kolloide, bei denen die disperse Phase (Kolloid) sich mit der kontinuierlichen Phase (Wasser) zu einem viskosen, gelartigen Produkt verbunden hat, z.B. koagulierte Siliziumsäure. Gegenüber anderen Materialien ergibt sich der Vorteil der Wasser-Abgabe unter Druck und der Wasser-Aufnahme bei Druckentlastung, d.h. analog zum natürlichen Nukleus.

Zweckmässigerweise wird der Körper spritzgusstechnisch hergestellt, wobei sein Anspritzpunkt vorzugsweise im Bereich des zweiten Endes positioniert ist. Dies bedeutet ist in der Produktion fülltechnisch von Vorteil. Der Anspritzpunkt ist vorzugsweise versenkt angeordnet. Die Verwerfungen, die durch das Abziehen der Nadeldüse entstehen, kommen dadurch nicht an der Oberfläche vor, sondern in der Ansenkung drin.

Bei einer speziellen Ausführungsform ist das erste Ende asymmetrisch zum Inneren der Spirale zulaufend ausgebildet. Dadurch ist die Aussenform des spiralförmig aufgewickelten Körpers abgerundet.

Der Körper kann röntgenopak ausgebildet werden, vorzugsweise durch einen Zusatz von Bariumsulfat. Damit wird eine Überprüfung der Lage des Implantates und die Kontrolle einer allfälligen Migration ermöglicht. Der Körper kann zum gleichen Zweck auch röntgenopake Elemente, vorzugsweise in Form von Filamenten, Drähten oder Kügelchen enthalten.

Bei einer speziellen Ausführungsform weist die letzte, äussere Spiralwindung von mindestens 360° Umfang des spiralförmig aufgewickelten Körpers - gegenüber den übrigen Spiralwindungen - einen dünneren Querschnitt auf. Der äussere Rand des Implantats ist dadurch flexibler bezogen auf die Funktion des Implantates.

Schliesslich kann das äussere Ende des Körpers mit Mitteln versehen sein, welche für die Erfassung der Bandscheibenprothese mittels eines Einführungsinstrumentes geeignet sind, vorzugsweise in Form von Vertiefungen oder Erhebungen.

Die Erfindung und Weiterbildungen der Erfindung werden im folgenden anhand der schematischen Darstellungen eines Ausführungsbeispiels noch näher erläutert.

Es zeigt
Fig. 1 eine perspektivische Ansicht einer erfindungsgemässen Bandscheibenprothese;
Fig. 2 zeigt einen horizontalen Querschnitt durch die Bandscheibenprothese nach Fig. 1;
Fig. 3 einen Querschnitt längs der Linie III-III in Fig. 2;
Fig. 4 eine Seitenansicht der Bandscheibenprothese nach Fig. 1;
Fig. 5 einen vergrösserten Ausschnitt in der Fig. 3 im Bereich des zentralen Anspritzpunktes;
Fig. 6 eine vergrösserte, perspektivische Ansicht des äusseren Endes des spiralförmig aufgewickelten Körpers gemäss Fig. 1; und
Fig. 7 eine Variante des äusseren Endes des spiralförmig aufgewickelten Körpers gemäss Fig. 1.

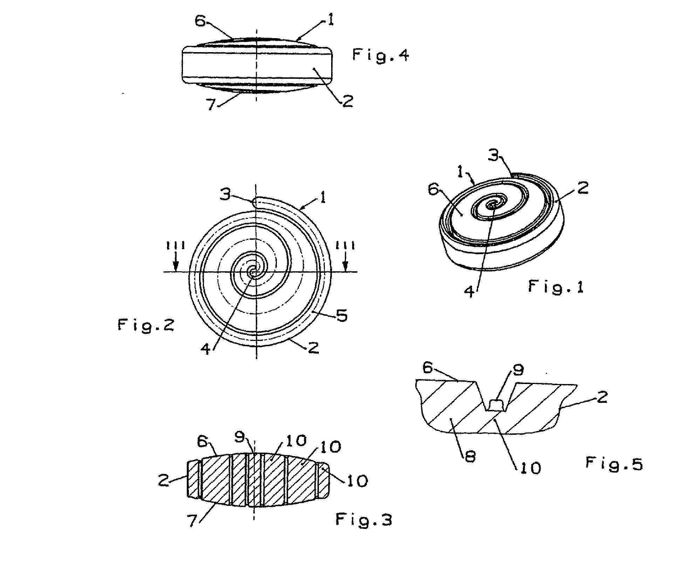

Die in den Fig. 1 bis 4 dargestellte Bandscheibenprothese 1 besteht aus einem longitudinalen, formelastischen, spiralförmig aufwickelbaren Körper 2 mit einem ersten, äusseren Ende 3, einem zweiten, inneren Ende 4 und einer longitudinalen Zentralachse 5. Der zur Zentralachse 5 orthogonale Querschnitt 10 des Körpers 2 verkleinert sich kontinuierlich - wie in den Fig. 2 und 3 dargestellt - sowohl gegen das zweite, innere Ende 4 hin als auch gegen das erste, äussere Ende 3 hin.

Die in der Fig. 2 gemessene Breite des Körpers 2 beträgt beim inneren Ende 4 etwa 2,5 mm, beim äusseren Ende 3 ebenfalls etwa 2,5 mm und wächst dazwischen gegen die Mitte des Körpers 2 bis auf etwa 4,5 mm an.

Die in der Fig. 3 gemessene Höhe der Bandscheibenprothese 1 entspricht dem anatomischen Zwischenwirbelraum. Die Höhe in der Mitte der konvexen Bandscheibenprothese 1 steht beidseitig um etwas 0,5 bis 3,0 mm vor im Vergleich zu den randständigen Partien.

Im spiralförmig aufgewickelten, nicht belasteten Zustand des Körpers 2 - wie er in Fig. 1 und 2 dargestellt ist - besteht zwischen den einzelnen Spiralwindungen ein Spalt von 0,65 mm.

Der Körper 2 besteht im wesentlichen aus einer Hülle aus Polycarbonaturethan und/oder aus Silikon-Polycarbonaturethan sowie einer Füllung aus einem Polyvinylalkohol-Hydrogel. Weitere geeignete Materialien, sowohl für die Hülle als auch für ihre Füllung, können der noch hängigen Internationalen Patentanmeldung PCT/CH01/00700 entnommen werden.

In Fig. 5 ist dargestellt, wie der Anspritzpunkt 9 im Bereich des zweiten Endes 4, d.h. annähernd im Zentrum des spiralförmigen Körpers 2, positioniert ist und gegenüber der oberen Spiralfläche 6 versenkt angeordnet ist.

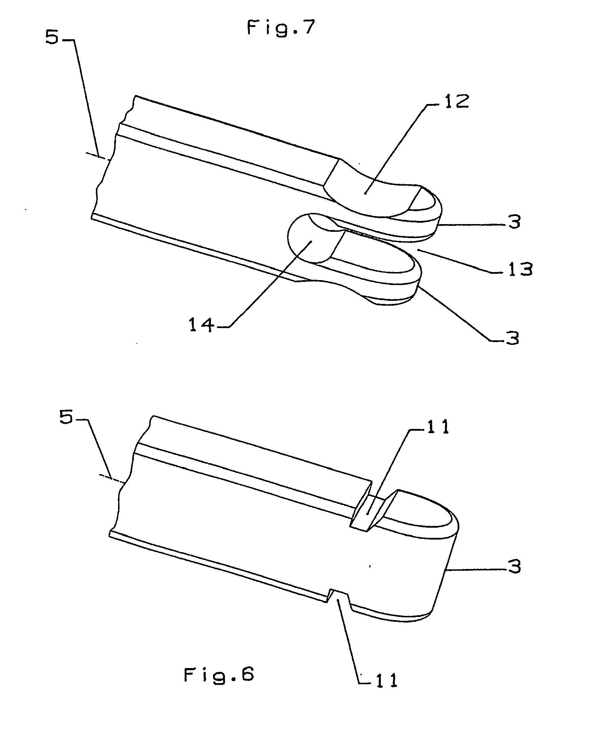

Die Fig. 6 zeigt eine mögliche Variante des äusseren Endes 3 des Körpers 2 mit Mitteln, in Form zweier quer zur Zentralachse 5 stehenden Nuten 11, welche eine einfache Erfassung der Bandscheibenprothese mittels eines geeigneten Einführungsinstrumentes, beispielsweise einer Zange gestattet.

Fig. 7 zeigt eine zweite Variante des äusseren Endes 3 des Körpers 2 mit Mitteln, hier in Form zweier quer zur Zentralachse 5 verlaufenden flachen Vertiefungen 12 sowie einem parallel zur Zentralachse 5 angeordneten Schlitz 13 mit einer zylindrischen Hinterschneidung 14.

## Patentansprüche

1. Bandscheibenprothese oder Nukleus-Ersatz-Prothese (1), bestehend aus einem longitudinalen, formelastischen, spiralförmig aufwickelbaren Körper (2), mit einem ersten, äusseren Ende (3), einem zweiten, inneren Ende (4) und einer longitudinalen Zentralachse (5),
**dadurch gekennzeichnet, dass**
der zur Zentralachse (5) orthogonale Querschnitt (10) des Körpers (2) sich gegen das zweite, innere Ende (4) hin verkleinert.

2. Bandscheibenprothese (1) nach Anspruch 1, **dadurch gekennzeichnet, dass** der orthogonale Querschnitt (10) sich kontinuierlich verkleinert.

3. Bandscheibenprothese (1) nach Anspruch 1 oder 2, **dadurch gekennzeichnet, dass** der zur Zentralachse (5) orthogonale Querschnitt (10) des Körpers (2) sich gegen das erste, äussere Ende (3) hin , vorzugsweise kontinuierlich verkleinert.

4. Bandscheibenprothese (1) nach einem der Ansprüche 1 bis 3, **dadurch gekennzeichnet, dass** sich die senkrecht zur Zentralachse (5) gemessene Breite des Körpers (2) - von seiner Mitte aus gesehen - gegen das äussere Ende (3) hin, vorzugsweise kontinuierlich verringert.

5. Bandscheibenprothese (1) nach einem der Ansprüche 1 bis 4, **dadurch gekennzeichnet, dass** sich die senkrecht zur Zentralachse (5) gemessene Breite des Körpers (2) - von seiner Mitte aus gesehen - gegen das innere Ende (4) hin, vorzugsweise kontinuierlich verringert.

6. Bandscheibenprothese (1) nach einem der Ansprüche 1 bis 5, **dadurch gekennzeichnet, dass** die Breite des Körpers (2) in seiner Mitte 50 % bis 500 %, vorzugsweise 100 % bis 300 % breiter ist als an seinem inneren und äusseren Ende (4,3).

7. Bandscheibenprothese (1) nach einem der Ansprüche 1 bis 6, **dadurch gekennzeichnet, dass** der Körper (2) im spiralförmig aufgewickelten Zustand zur Zentralachse (5) eine obere Spiralfläche (6) und eine untere Spiralfläche (7) aufweist, welche beide konvex gewölbt und zur Anlage an die Deckplatten zweier benachbarter Wirbelkörper geeignet sind.

8. Bandscheibenprothese (1) nach einem der Ansprüche 1 bis 7, **dadurch gekennzeichnet, dass** der Körper (2) im spiralförmig aufgewickelten, nicht belasteten Zustand zwischen den Spiralen einen Spalt aufweist.

9. Bandscheibenprothese (1) nach Anspruch 8, **dadurch gekennzeichnet, dass** der Spalt eine Breite von mindestens 0,4 mm, vorzugsweise mindestens 0,5 mm aufweist.

10. Bandscheibenprothese (1) nach Anspruch 8 oder 9, **dadurch gekennzeichnet, dass** der Spalt eine Breite von höchstens 1,0 mm, vorzugsweise von höchstens 0,8 mm aufweist.

11. Bandscheibenprothese (1) nach einem der Ansprüche 1 bis 10, **dadurch gekennzeichnet, dass** der Körper (2) im spiralförmig aufgewickelten Zustand, in der Spiralebene gesehen eine ovale oder nierenförmige Gestalt aufweist, vorzugsweise mit einer in der Spiralebene gemessenen Fläche von 250 bis 750 mm².

12. Bandscheibenprothese (1) nach einem der Ansprüche 1 bis 11, **dadurch gekennzeichnet, dass** der Körper (2) ein Hydrogel enthält und vorzugsweise vollständig aus einem Hydrogel besteht.

13. Bandscheibenprothese (1) nach einem der Ansprüche 1 bis 9, **dadurch gekennzeichnet, dass** der Körper (2) spritzgusstechnisch hergestellt ist und sein Anspritzpunkt (9) im Bereich des zweiten Endes (4) positioniert ist.

14. Bandscheibenprothese (1) nach Anspruch 13, **dadurch gekennzeichnet, dass** der Anspritzpunkt (9) gegenüber der oberen Spiralfläche (6) versenkt angeordnet ist.

15. Bandscheibenprothese (1) nach einem der Ansprüche 1 bis 14, **dadurch gekennzeichnet, dass** das erste Ende (3) asymmetrisch zum Inneren der Spirale zulaufend ausgebildet ist.

16. Bandscheibenprothese (1) nach einem der Ansprüche 1 bis 15, **dadurch gekennzeichnet, dass** der Körper (2) röntgenopak ist, vorzugsweise erzeugt durch einen Zusatz von Bariumsulfat.

17. Bandscheibenprothese (1) nach einem der Ansprüche 1 bis 16, **dadurch gekennzeichnet, dass** der Körper (2) röntgenopake Elemente, vorzugsweise in Form von Filamenten, Drähten oder Kügelchen enthält.

18. Bandscheibenprothese (1) nach einem der Ansprüche 1 bis 17, **dadurch gekennzeichnet, dass** die letzte, äussere Spiralwindung von mindestens 360° Umfang des spiralförmig aufgewickelten Körpers (2) gegenüber den übrigen Spiralwindungen einen dünneren Querschnitt aufweist.

19. Bandscheibenprothese (1) nach einem der Ansprüche 1 bis 18, **dadurch gekennzeichnet, dass** das äusseren Ende (3) des Körpers (2) mit Mitteln (11;12,13,14) versehen ist, welche für die Erfassung der Bandscheibenprothese (1) mittels eines Einführungsinstrumentes geeignet sind, vorzugsweise in Form von Vertiefungen oder Erhebungen.

## Claims

1. Intervertebral disk prosthesis or nucleus replacement prosthesis (1) consisting of a longitudinal body body (2) which has an elasticity of shape and can be wound in a spiral shape, with a first exterior end (3), a second interior end (4) and a longitudinal central axis (5),
**characterised in that**
the cross-section (10) of the body (2) which is orthogonal towards the central axis (5) is reduced in size towards the second interior end (4).

2. Intervertebral disk prosthesis (1) as per claim 1 **characterised in that** the orthogonal cross-section (10) is continuously reduced in size.

3. Intervertebral disk prosthesis (1) as per claim 1 or 2 **characterised in that** the cross-section (10) of the body (2), which is orthogonal towards the central axis (5), is reduced in size towards the first exterior end (3), preferably with the reduction occurring continuously.

4. Intervertebral disk prosthesis (1) as per one of the claims 1 to 3 **characterised in that** the width of the body (2), measured vertically towards the central axis (5), is reduced in size - viewed from its centre - towards the exterior end (3), preferably with the reduction occurring continuously.

5. lntervertebral disk prosthesis (1) as per one of the claims 1 to 4 **characterised in that** the width of the body (2), measured vertically towards the central axis (5), is reduced in size - viewed from its centre - towards the interior end (4), preferably with the reduction occurring continuously.

6. Intervertebral disk prosthesis (1) as per one of the claims 1 to 5 **characterised in that** the width of the body (2) in its centre is wider by 50% to 500%, preferably wider by 100% to 300%, than at its interior and exterior ends (4,3).

7. Intervertebral disk prosthesis (1) as per one of the claims 1 to 6 **characterised in that** the body (2) in its spiral-wound state features an upper spiral level (6) and a lower spiral level (7) with respect to the central axis (5), which are both curved in convex shapes and are suitable for application to the cover plates of two adjacent intervertebral disk spaces.

8. Intervertebral disk prosthesis (1) as per one of the claims 1 to 7 **characterised in that** body (2) in a spiral-wound unloaded state features a gap between the spirals.

9. Intervertebral disk prosthesis (1) as per claim 8 **characterised in that** the gap has a minimum width of 0,4 mm, preferably a minimum of 0,5 mm.

10. Intervertebral disk prosthesis (1) as per claim 8 or 9 **characterised in that** the gap has a maximum width of 1,0 mm, preferably a maximum of 0,8 mm.

11. Intervertebral disk prosthesis (1) as per one of the claims 1 to 10 **characterised in that** the body (2) in a spiral-wound state - viewed at the spiral level - features an oval or kidney-shaped shape, preferably with a surface between 250 to 750 mm² measured at the spiral level.

12. Intervertebral disk prosthesis (1) as per one of the claims 1 to 11 **characterised in that** body (2) contains a hydrogel and preferably consists completely of hydrogel.

13. Intervertebral disk prosthesis (1) as per one of the claims 1 to 9 **characterised in that** body (2) is manufactured using an injection-molding process and its injection point (9) is positioned in the area of the second end (4).

14. Intervertebral disk prosthesis (1) as per claim 13 **characterised in that** the injection point (9) is located in a recess vis-à-vis the upper spiral level (6).

15. Intervertebral disk prosthesis (1) as per one of the claims 1 to 14 **characterised in that** the first end (3) is designed asymmetrically towards the interior of the spiral.

16. Intervertebral disk prosthesis (1) as per one of the claims 1 to 15 **characterised in that** body (2) is X-ray-opaque, preferably achieved by using an addition of barium sulfate.

17. Intervertebral disk prosthesis (1) as per one of the claims 1 to 16 **characterised in that** body (2) contains X-ray-opaque components, preferably in the form of filaments, wires or tiny globes.

18. Intervertebral disk prosthesis (1) as per one of the claims 1 to 17 **characterised in that** the last exterior spiral turn with a circumference of at least 360° of the spiral-wound body (2) features a thinner cross-section when compared with the remaining spiral turns.

19. Intervertebral disk prosthesis (1) as per one of the claims 1 to 18 **characterised in that** the exterior end (3) of the body (2) is fitted with means (11, 12, 13, 14), which are suited for gripping the intervertebral disk prosthesis (1) using an insertion instrument, preferably in the form of indentations or protrusions.

## Revendications

1. Prothèse de disque intervertébral ou prothèse de remplacement du nucléus (1), formée d'un corps longitudinal (2) à élasticité de forme, pouvant être enroulé en spirale, comprenant une première extrémité externe (3), une deuxième extrémité interne (4) et un axe central longitudinal (5),
**caractérisée en ce que**
la section (10) du corps (2), orthogonale à l'axe central (5), décroît en direction de la deuxième extrémité interne (4).

2. Prothèse de disque intervertébral (1) selon la revendication 1, **caractérisée en ce que** la section orthogonale (10) décroît en continu.

3. Prothèse de disque intervertébral (1) selon la revendication 1 ou 2, **caractérisée en ce que** la section (10) du corps (2), orthogonale à l'axe central (5), décroît en direction de la première extrémité externe (3), de préférence en continu.

4. Prothèse de disque intervertébral (1) selon l'une quelconque des revendications 1 à 3, **caractérisée en ce que** la largeur du corps (2) mesurée perpendiculairement à l'axe central (5) - vue depuis son centre - décroît en direction de l'extrémité externe (3), de préférence en continu.

5. Prothèse de disque intervertébral (1) selon l'une quelconque des revendications 1 à 4, **caractérisée en ce que** la largeur du corps (2) mesurée perpendiculairement à l'axe central (5) - vue depuis son centre - décroît en direction de l'extrémité interne (4), de préférence en continu.

6. Prothèse de disque intervertébral (1) selon l'une quelconque des revendications 1 à 5, **caractérisée en ce que** la largeur du corps (2), au centre de celui-ci, est 50% à 500% plus large, de préférence 100% à 300% plus large qu'au niveau de ses extrémités interne et externe (4, 3).

7. Prothèse de disque intervertébral (1) selon l'une quelconque des revendications 1 à 6, **caractérisée en ce qu'**à l'état enroulé en spirale, le corps (2) présente, par rapport à l'axe central (5), une surface de spirale supérieure (6) et une surface de spirale inférieure (7), qui sont toutes deux convexes et appropriées pour s'appuyer sur les plaques de recouvrement de deux corps vertébraux adjacents.

8. Prothèse de disque intervertébral (1) selon l'une quelconque des revendications 1 à 7, **caractérisée en ce qu'**à l'état enroulé en spirale non sollicité, le corps (2) présente une fente entre les spirales.

9. Prothèse de disque intervertébral (1) selon la revendication 8, **caractérisée en ce que** la fente présente une largeur d'au moins 0,4 mm, de préférence d'au moins 0,5 mm.

10. Prothèse de disque intervertébral (1) selon la revendication 8 ou 9, **caractérisée en ce que** la fente présente une largeur d'au maximum 1,0 mm, de préférence d'au maximum 0,8 mm.

11. Prothèse de disque intervertébral (1) selon l'une quelconque des revendications 1 à 10, **caractérisée en ce qu'**à l'état enroulé en spirale, vu dans le plan de la spirale, le corps (2) présente une forme ovale ou réniforme, de préférence avec une surface de 250 à 750 mm² mesurée dans le plan de la spirale.

12. Prothèse de disque intervertébral (1) selon l'une quelconque des revendications 1 à 11, **caractérisée en ce que** le corps (2) contient un hydrogel et se compose de préférence entièrement d'un hydrogel.

13. Prothèse de disque intervertébral (1) selon l'une quelconque des revendications 1 à 9, **caractérisée en ce que** le corps (2) est fabriqué selon la technique du moulage par injection et son point d'injection (9) se trouve au niveau de la deuxième extrémité (4).

14. Prothèse de disque intervertébral (1) selon la revendication 13, **caractérisée en ce que** le point d'injection (9) est disposé dans un renfoncement par rapport à la surface de spirale supérieure (6).

15. Prothèse de disque intervertébral (1) selon l'une quelconque des revendications 1 à 14, **caractérisée en ce que** la première extrémité (3) présente une forme asymétrique en direction de l'intérieur de la spirale.

16. Prothèse de disque intervertébral (1) selon l'une quelconque des revendications 1 à 15, **caractérisée en ce que** le corps (2) est opaque aux rayons X, de préférence grâce à une addition de sulfate de baryum.

17. Prothèse de disque intervertébral (1) selon l'une quelconque des revendications 1 à 16, **caractérisée en ce que** le corps (2) contient des éléments opaques aux rayons X, de préférence sous la forme de filaments, de fils ou de billes.

18. Prothèse de disque intervertébral (1) selon l'une quelconque des revendications 1 à 17, **caractérisée en ce que** le dernier enroulement externe de la spirale d'au moins 360° du corps enroulé en spirale (2) présente une section plus mince par rapport aux autres enroulements de la spirale.

19. Prothèse de disque intervertébral (1) selon l'une quelconque des revendications 1 à 18, **caractérisée en ce que** l'extrémité externe (3) du corps (2) est pourvue de moyens (11, 12, 13, 14), qui sont appropriés pour saisir la prothèse de disque intervertébral (1) au moyen d'un instrument d'introduction, de préférence sous la forme de creux ou de bosses.
